# FASCICULE DE BREVET EUROPEEN

(11) **EP 4 444 702 B1**
(45) Date de publication et mention de la délivrance du brevet: **17.12.2025**
(21) Numéro de dépôt: 22835463.5
(22) Date de dépôt: 05.12.2022
(51) Int. Cl.: C07D 233/08, B01D 3/00, C07B 45/00

(54) **PROCÉDÉ DE PRODUCTION DE TÉTRAHYDROTHIOPHÈNE**
VERFAHREN ZUR HERSTELLUNG VON TETRAHYDROTHIOPHEN
METHOD FOR PRODUCING TETRAHYDROTHIOPHENE

(30) Priorité: 06.12.2021 FR 2112992
(43) Date de publication de la demande: 16.10.2024
(73) Titulaire: ARKEMA FRANCE, 92800 Puteaux (FR)
(72) Inventeur: FREMY, Georges, 64170 LACQ (FR); LAMANT, Eric, 64170 LACQ (FR); RAYMOND, Jean-Michel, 40300 CAUNEILLE (FR)
(74) Mandataire: Arkema Patent
(86) Numéro de dépôt international: PCT/FR2022/052245
(87) Numéro de publication internationale: WO 2023/105151

(56) Documents cités:
- CN-A- 105 949 171
- US-A- 3 819 651

## Description

La présente invention concerne un procédé de production de tétrahydrothiophène intégrant le traitement des rejets gazeux. La présente invention concerne également un procédé de traitement des rejets gazeux d'une unité de production de tétrahydrothiophène.

Le tétrahydrothiophène est un composé présentant un grand intérêt industriel. Il est connu pour être utilisé comme additif dans les gaz consommables pour déceler d'éventuelles fuites en raison de son odeur très caractéristique. De ce fait, il est présent dans le gaz injecté sur le réseau d'alimentation domestique, dans les réseaux de transport de gaz, dans les appareils de stockage et dans les interconnexions avec les autres réseaux de transport de gaz.

Il est connu de synthétiser le tétrahydrothiophène, ci-après noté THT, à partir de 1,4-butanediol, ci-après noté BDO ou bien de tétrahydrofurane, noté THF, et de sulfure d'hydrogène, ci-après noté H₂S en présence de catalyseur hétérogène (CN105949171, US3819651).

Il est admis dans la littérature que la première étape *in situ* est la transformation du BDO en THF selon la réaction suivante : 1,4-BDO →THF + H₂O

La deuxième étape est la transformation du THF, en présence de H₂S, en THT selon la réaction suivante :

THF + H₂S →THT + H₂O

Or, les réactions secondaires suivantes peuvent se produire en fonction des conditions opératoires :

THT →DiHydroThiophène + H₂

THT →Thiophène + 2H₂

THT + H₂ →Propylène + CH₃SH

THT + H₂ →Butène + H₂S

De plus, cette synthèse peut générer des produits de dégradation, tels que du CO₂, de l'éthylène et des mercaptans en C₁ à C₄.

Par ailleurs, des rejets gazeux, également appelés évents gazeux, sont émis tout au long du procédé de production. Ces rejets peuvent contenir l'excès de sulfure d'hydrogène éventuellement introduit dans le circuit, des produits de craquage, des composés intermédiaires tels que le THF, et également des quantités significatives de THT. Habituellement, ces rejets sont incinérés, conduisant à de fortes émissions d'oxydes de soufre (SO₂) dans l'atmosphère, potentiellement responsables de pluies acides. Or, à l'heure actuelle, ces rejets ne sont plus tolérés. Il est à noter que, ces rejets gazeux ne peuvent pas être recyclés tels quels dans la réaction principale, car la présence d'éthylène, de propylène et de butène conduit à un vieillissement très prématuré du catalyseur par la formation de gommes et de coke. Ainsi, pour être recyclés, ces rejets doivent subir une purge très conséquente pour ne pas recycler de quantités importantes d'hydrocarbures en C₂ à C₄. Or, cette purge a également pour conséquence de perdre la quasi-totalité du THT et des composés soufrés ou oxygénés présents dans ces évents.

Or, au regard des considérations écologiques actuelles, il existe aujourd'hui un réel besoin pour un procédé de synthèse de THT plus respectueux de l'environnement, tout en conservant des rendements élevés.

Un objectif de la présente invention est de fournir un procédé de préparation de THT permettant une gestion améliorée des rejets gazeux, et notamment qui soit plus respectueux de l'environnement.

Un autre objectif de la présente invention est de diminuer les rejets gazeux, et notamment la quantité de dioxyde de soufre, émis lors de la production de tétrahydrothiophène, après incinération des rejets.

Un autre objectif de la présente invention est de fournir un procédé de préparation de tétrahydrothiophène plus économique.

Enfin, un autre objectif de la présente invention est de fournir un procédé et/ou un dispositif de traitement des rejets gazeux qui soit(soient) facilement intégrable(s) dans une unité de production de tétrahydrothiophène.

Le procédé selon l'invention répond en tout ou partie aux objectifs mentionnés ci-dessus.

Les présents inventeurs ont découvert de façon surprenante que les rejets gazeux peuvent être récupérés et traités par une extraction gaz-liquide, avec le 1,4-butanediol en tant que solvant de l'extraction. L'extraction gaz(évents)-liquide(1,4-butanediol) selon l'invention permet notamment de faire passer les composés soufrés, ainsi que le tétrahydrofurane contenus dans lesdits évents gazeux dans le 1,4-butanediol liquide.

Par « composés soufrés », on entend des composés qui comprennent au moins un atome de soufre, de préférence un atome de soufre. En particulier, par « composés soufrés », on entend le tétrahydrothiophène et des sous-produits soufrés comme le thiophène, le dihydrothiophène, des mercaptans, et éventuellement le sulfure d'hydrogène, ces composés ayant été entrainés dans les évents gazeux lors de la ou des étapes de purification, de préférence dans les étapes de condensation, et/ou de décantation et/ou de distillation, après l'étape a).

Ainsi, l'extraction gaz-liquide selon l'invention permet de récupérer les composés soufrés, et notamment le tétrahydrothiophène, ainsi que le tétrahydrofurane, qui est un intermédiaire de la réaction.

Le procédé selon l'invention utilise un des réactifs de synthèse, c'est-à-dire le 1,4-butanediol en tant que solvant de l'étape d'extraction gaz-liquide. Ainsi, la récupération des composés soufrés et du tétrahydrofurane utilise le réactif de synthèse, optimisant ainsi cette voie de recyclage.

De façon très avantageuse, le 1,4-butanediol enrichi en composé(s) soufré(s) et éventuellement en tétrahydrofurane est utilisé, de préférence directement (par exemple sans étape de purification), en tant que réactif pour former le tétrahydrothiophène. Ainsi, le procédé selon la présente invention permet de réintroduire dans le procédé de production de tétrahydrothiophène des composés d'intérêt, qui étaient jusqu'alors incinérés.

L'étape d'extraction selon l'invention présente l'avantage d'être facilement intégrable à une unité de production de tétrahydrothiophène, car elle traite uniquement les évents gazeux. Elle consomme donc peu d'énergie et utilise un dispositif simple.

Ladite extraction permet également de diminuer la quantité des évents à traiter par incinération et permet de diminuer fortement le relargage de dioxyde de soufre dans l'atmosphère.

Le procédé de production de tétrahydrothiophène selon l'invention est donc plus économique, présente une meilleure productivité, tout en étant plus respectueux de l'environnement.

### Brève description de l'invention

Ainsi, la présente invention a pour objet un procédé de production de tétrahydrothiophène comprenant les étapes successives suivantes :
a) réaction de 1,4-butanediol en présence de sulfure d'hydrogène (H₂S), en phase gaz, en présence d'au moins un catalyseur pour former un flux (A) comprenant du tétrahydrothiophène, de l'eau, et éventuellement du sulfure d'hydrogène n'ayant pas réagi ;
b) condensation du flux (A) pour obtenir un flux (B) riche en tétrahydrothiophène et des évents gazeux (C) contenant les incondensables éventuellement formés à l'issue de l'étape a) ;
c) au moins une étape de purification, de préférence par décantation, du flux (B) avec séparation de la phase aqueuse, de la phase organique constituant le flux (D) et des évents gazeux (E) ;
d) éventuellement au moins une distillation du flux (D) pour isoler le tétrahydrothiophène des évents gazeux (F) ;
e) récupération du tétrahydrothiophène isolé à l'étape c) et éventuellement à l'étape d),
   les étapes b) et c) pouvant être successives ou simultanées,
f) extraction gaz-liquide à l'aide d'une colonne, alimentée par un ou plusieurs flux gazeux (G) comprenant les évents gazeux (C) et/ou les évents gazeux (E) et éventuellement les évents gazeux (F) et par un flux liquide de 1,4-butanediol, pour former en sortie de colonne un flux gazeux (H) et un flux liquide (I) comprenant du 1,4-butanediol enrichi ;
g) recyclage de tout ou partie du flux (I) vers la réaction a).

### Brève description de la figure

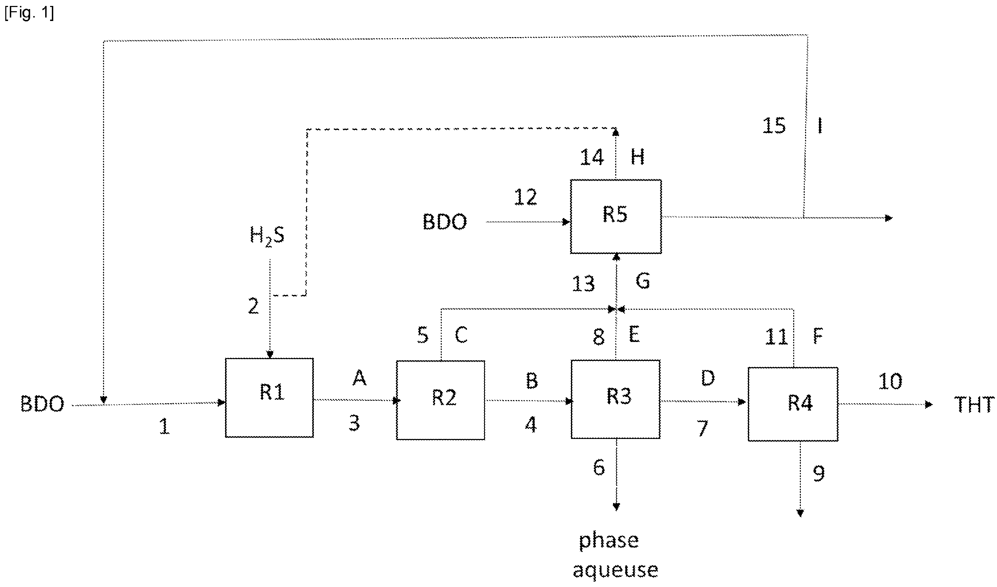

[Fig. 1] est un schéma du dispositif mettant en œuvre le procédé revendiqué.

### Description détaillée de l'invention

D'autres caractéristiques, aspects, objets et avantages de la présente invention apparaîtront encore plus clairement à la lecture de la description qui suit.

Il est précisé que les expressions « de ...à ... » et « compris entre ... et .... » utilisées dans la présente description doivent s'entendre comme incluant chacune des bornes mentionnées.

On entend par « 1,4-butanediol enrichi », le 1,4-butanediol obtenu après l'extraction gaz-liquide selon l'invention, c'est-à-dire obtenu après l'étape f).

En particulier, le 1,4-butanediol enrichi est une composition comprenant du 1,4-butanediol et au moins un composé soufré, de préférence comprenant du 1,4-butanediol et du tétrahydrothiophène, et éventuellement des sous-produits soufrés tels que le thiophène et le dihydrothiophène, et éventuellement du tétrahydrofurane. En particulier, ledit 1,4-butanediol enrichi comprend entre 0,1% et 20% en poids de tétrahydrothiophène, de préférence entre 1% et 10%, plus préférentiellement entre 1% et 5% en poids de tétrahydrothiophène, par rapport au poids total du 1,4-butanediol enrichi.

Plus particulièrement, on entend par « 1,4-butanediol enrichi », une composition comprenant :
- du 1,4-butanediol, de préférence au moins 50% en poids de 1,4-butanediol, plus préférentiellement au moins 80% en poids, encore préférentiellement au moins 90% en poids de 1,4-butanediol, par rapport au poids total de la composition ;
- du tétrahydrothiophène, de préférence entre 0,1% et 20% en poids de tétrahydrothiophène, plus préférentiellement entre 1% et 10%, encore plus préférentiellement entre 1% et 5% en poids de tétrahydrothiophène, par rapport au poids total de la composition ;
- éventuellement des sous-produits soufrés, de préférence le dihydrothiophène et/ou le thiophène ;
- éventuellement du tétrahydrofurane ;
- éventuellement du sulfure d'hydrogène ;
- éventuellement de l'eau ; et
- éventuellement des composés incondensables.

Ladite composition peut ainsi comprendre :
- entre 80% et 99% en poids de 1,4-butanediol par rapport au poids total de la composition et
- entre 0,1% et 10%, de préférence entre 1% et 5%, en poids de tétrahydrothiophène par rapport au poids total de la composition.

Par « évent ou rejet gazeux », on entend une phase gazeuse comprenant au moins un composé soufré tel que défini ci-dessus et notamment récupérée suite à au moins une étape de séparation ou de purification du flux (A), qui fait suite à la réaction a).

Lesdits évents gazeux, et notamment les flux (C), (E) et (F) comprennent au moins un composé soufré et éventuellement du tétrahydrofurane.

Lesdits évents gazeux peuvent comprendre, voire être constitués de tétrahydrothiophène, de tétrahydrofurane, de composés incondensables, d'eau et de sous-produits soufrés, éventuellement de sulfure d'hydrogène.

Lesdits évents peuvent être issus d'un condenseur, d'un décanteur et/ou du flux de tête de la colonne de distillation et/ou d'une purge.

En particulier, les évents ou rejets gazeux selon l'invention sont normalement considérés comme des déchets et sont habituellement envoyés à l'incinérateur.

On entend par « composés incondensables », des composés qui restent à l'état gazeux dans les conditions des étapes du procédé. On peut citer comme composés incondensables CH₄, CO, CO₂, H₂ et N₂.

Le procédé selon l'invention comprend les étapes consécutives précitées : étapes a) à g). Ce procédé peut comporter des étapes de purification intermédiaires.

### Etape a) - Réaction

Lors de l'étape a), on fait réagir du 1,4-butanediol avec du sulfure d'hydrogène pour former un flux (A), de préférence à l'état gazeux, comprenant du tétrahydrothiophène, de l'eau, éventuellement le sulfure d'hydrogène n'ayant pas réagi, éventuellement du tétrahydrofurane, et éventuellement des sous-produits soufrés. Le flux (A) peut contenir également du 1,4-butanediol n'ayant pas réagi. Des oléfines et/ou des incondensables peuvent également être présents dans le flux (A).

Ainsi, des flux gazeux de sulfure d'hydrogène et de 1,4-butanediol, chacun sous forme gazeuse, sont introduits dans un réacteur. Ces réactifs peuvent arriver dans le réacteur via un même flux ou bien de manière séparée. De préférence, les réactifs arrivent via un même flux, le flux de sulfure d'hydrogène étant préalablement chauffé et permettant de vaporiser le flux liquide de 1,4-butanediol.

La réaction se fait en phase gaz et de préférence en continu. Le ou les catalyseurs peuvent être en lit fixe dans un réacteur multitubulaire ou non, isotherme ou adiabatique, à plaques. Un réacteur multizone ou plusieurs réacteurs en série avec des catalyseurs identiques ou différents avec multi-injection de BDO ou non, peuvent également être utilisés.

La température de réaction peut être comprise entre 200°C et 450°C, de préférence entre 200°C et 380°C. De préférence, la température de réaction est comprise entre 200°C et 360°C. Au-dessus de cette température, le catalyseur peut être physiquement endommagé (par frittage et cokage notamment). Cette température peut évoluer en fonction de la durée de fonctionnement du catalyseur, qui peut se désactiver partiellement dans le temps.

La pression dans le réacteur peut aller de la pression atmosphérique à 50 bars, de préférence de 1 à 30 bars, et de préférence encore de 1 à 10 bars.

Le rapport molaire de sulfure d'hydrogène/1,4-butanediol peut être compris entre 1 et 100, de préférence entre 1 et 50, plus particulièrement entre 1 et 10. Le sulfure d'hydrogène est de préférence en excès par rapport au 1,4-butanediol.

Le débit de BDO par rapport à la quantité de catalyseur peut être compris entre 0,005 et 10 kg/h/kg de catalyseur, de préférence entre 0,1 et 5 kg/h/kg, encore de préférence entre 0,1 et 1 kg de BDO kg/h/kg de catalyseur.

Le réacteur contient un catalyseur pour la réaction de formation du tétrahydrothiophène, de préférence en phase gaz. Parmi les catalyseurs pouvant être utilisés, on peut utiliser les silices, les alumines, les silico-aluminates. De préférence, les catalyseurs sont des alumines plus ou moins dopées avec des oxydes d'alcalins ou d'alcalino-terreux, de préférence non dopées.

De préférence, le catalyseur utilisé est une alumine, contenant une teneur en Na₂O inférieure à 0,3% en poids par rapport au poids total du catalyseur, telle que la sphéralite 505 commercialisée par Axens.

On obtient ainsi un flux (A) comprenant du tétrahydrothiophène, de l'eau, éventuellement du sulfure d'hydrogène n'ayant pas réagi, éventuellement du tétrahydrofurane, et éventuellement des sous-produits soufrés.

### Etape b) - Condensation

Le flux (A) issu de l'étape a) subit une étape de condensation à l'aide de toute technique classique, de préférence à l'aide d'un ou plusieurs condenseur(s) ou économiseur(s). Lors de la condensation, le flux (A) est notamment refroidi aussi bas que possible, pour maximiser l'élimination de l'eau. De préférence, le flux (A) est condensé à une température comprise entre 20°C et 70°C, par exemple entre 30°C et 60°C. L'étape de condensation permet d'obtenir un flux (B) riche en tétrahydrothiophène et des évents gazeux (C) contenant les incondensables éventuellement formés à l'issue de l'étape a) et contenant au moins un composé soufré et éventuellement du tétrahydrofurane. De préférence, les évents gazeux (C) comprend du tétrahydrothiophène, les incondensables éventuellement formés à l'issue de l'étape a), éventuellement le sulfure d'hydrogène n'ayant pas réagi, éventuellement du tétrahydrofurane, et éventuellement des sous-produits soufrés.

### Etape c) - Purification, de préférence par décantation

Le flux (B) riche en tétrahydrothiophène subit une étape de purification. De préférence, il s'agit d'une étape de décantation de manière à séparer la phase organique de la phase aqueuse contenant l'eau générée au cours de la réaction a). De plus, au cours de cette étape, des évents gazeux sont également éliminés. Ainsi, la phase organique (D) comportant le tétrahydrothiophène est séparée de la phase aqueuse. Les évents gazeux sont récupérés dans un flux gazeux noté (E), qui comprend au moins un composé soufré et éventuellement du tétrahydrofurane. De préférence, le flux gazeux (E) comprend du tétrahydrothiophène, éventuellement le sulfure d'hydrogène n'ayant pas réagi, éventuellement du tétrahydrofurane, et éventuellement des sous-produits soufrés.

De préférence, le flux (B) est séparé à une température comprise entre 20°C et 70°C, de préférence, entre 30°C et 60°C. La pression peut être comprise entre 1 et 40 bars absolus.

### Etape d) - Eventuelle distillation

Le flux (D), c'est-à-dire la phase organique issue de l'étape de purification c) peut subir une ou plusieurs distillations. Cette ou ces distillations successives permettent d'éliminer les composés plus lourds que le THT, et/ou les composés plus légers que le THT.

Selon un mode de réalisation de l'invention, deux colonnes de distillation peuvent être utilisées en série, la première pour séparer les composés lourds, et la seconde pour séparer les composés légers.

Selon un autre mode de réalisation de l'étape de distillation, la distillation est réalisée avec une colonne à cloison de type DWC, comportant 3 sorties : la fraction de tête comportant les impuretés légères, la fraction en queue comportant les impuretés lourdes et la fraction intermédiaire comportant le THT purifié.

La distillation de l'étape d) peut être effectuée à une pression comprise entre 0,05 et 75 bars absolus, de préférence entre 1 et 30 bars absolus, plus préférentiellement entre 1 et 5 bars absolus.

La distillation de l'étape d) peut être effectuée à une température comprise entre 20°C et 200°C, de préférence entre 40°C et 160°C, plus préférentiellement entre 60°C et 140°C.

La distillation peut être réalisée dans n'importe quel type de colonne à distiller connue. Il peut s'agir d'une colonne à plateaux (par exemple plateaux à calottes, plateaux à soupapes ou plateaux perforés) ou à garnissage (par exemple à garnissage en vrac ou structuré). La distillation peut être réalisée dans une colonne à plateaux, de préférence comprenant entre 5 et 50 plateaux, plus préférentiellement entre 10 et 40 plateaux, par exemple entre 25 et 30 plateaux. La distillation peut également être effectuée dans une colonne à cloison (appelée DWC en langue anglaise pour Divided Wall Column). La cloison peut être fixe ou mobile, par exemple avec un garnissage structuré ou vrac.

### Etape e) récupération du tétrahydrothiophène isolé à l'étape c) et/ou d)

Le tétrahydrothiophène est récupéré à l'issue de l'étape c) et/ou d) pour pouvoir être stocké ou bien pour pouvoir être engagé directement dans une autre étape de synthèse.

### Etape f) - Extraction liquide-gaz

L'étape d'extraction liquide-gaz a pour but de récupérer les évents gazeux générés après l'étape de réaction a). Il est possible de récupérer un seul flux gazeux ou bien la totalité des flux gazeux de l'unité de production.

De préférence, les évents gazeux contenant les incondensables (C) issus de l'étape de condensation b), les évents gazeux (E) issus de l'étape de purification c) et éventuellement la fraction gazeuse (F) issue de l'étape de distillation d) peuvent être récupérés et réunis en un flux (G). On ne sortirait pas du cadre de l'invention si les flux (C), (E) et (F) arrivaient séparément dans l'unité d'extraction liquide-gaz.

L'étape d'extraction liquide-gaz est réalisée au sein d'une unité comportant au moins une colonne. Celle-ci est alimentée par un ou plusieurs flux gazeux et par un flux liquide de 1,4-butanediol. En sortie de colonne, un flux gazeux (H) est généré comportant des composés incondensables, et éventuellement du sulfure d'hydrogène, et un flux liquide (I) comprenant du 1,4-butanediol enrichi.

L'extraction gaz-liquide peut être effectuée dans au moins une colonne d'absorption ou dans au moins une cuve, de préférence à agitation mécanique. Ladite(lesdites) colonne(s) d'absorption est(sont) notamment choisie(s) parmi les colonnes à garnissage (par exemple garnissage vrac ou structuré), les colonnes à plateaux, les colonnes à bulles, les colonnes à pulvérisation et les colonnes à film tombant, voire des cuves à agitation mécanique. De préférence, on utilise une(des) colonne(s) à garnissage, par exemple entre 1 et 10 colonnes. Plusieurs colonnes d'absorption peuvent être utilisées, en parallèle ou en série. De préférence, l'extraction gaz-liquide est effectuée dans au moins une colonne d'absorption ou dans au moins une cuve, de préférence à agitation mécanique.

Les débits des phases gaz (évents) et liquide (1,4-butanediol) dépendent du type et du nombre de colonnes. Les évents gazeux et le 1,4-butanediol liquide arrivent dans la(les) colonne(s) d'absorption à co-courant ou à contre-courant, de préférence à contre-courant. Par exemple, les évents gazeux arrivent par le bas de la(des) colonne(s) et le 1,4-butanediol liquide par le haut de la(des) colonne(s), avec ou sans recirculation de la phase liquide sur la colonne.

Ce type de dispositif permettant de réaliser une extraction gaz-liquide est généralement appelé « absorbeur », et dans le cas de la présente invention, « absorbeur au 1,4-butanediol ».

L'extraction gaz-liquide peut être réalisée à une température comprise entre 25°C et 200°C, de préférence entre 25°C et 150°C, de préférence entre 25°C et 100°C, plus préférentiellement entre 40°C et 90°C. L'extraction gaz-liquide est réalisée à une pression comprise entre 1 et 40 bars absolus, de préférence entre 1 et 10 bars absolus, plus préférentiellement encore entre 1 et 5 bars absolus.

Le ratio massique des évents gazeux par rapport au 1,4-butanediol peut être compris entre 0,001 et 10, de préférence entre 0,1 et 3, de manière encore préférée entre 0,5 et 2.

L'étape f) permet de faire passer les composés soufrés, et éventuellement le tétrahydrofurane, dans le 1,4-butanediol liquide et de diminuer, voire d'éviter après incinération, les rejets de SO₂ dans l'atmosphère.

Les évents gazeux ainsi traités (c'est-à-dire dont au moins un composé soufré a été absorbé dans le 1,4-butanediol) peuvent être ensuite récupérés, éventuellement incinérés, et relargués après incinération dans l'atmosphère avec une teneur en SO₂ diminuée, de préférence lesdits évents après incinération ne comprennent quasiment pas ou ne comprennent pas de SO₂.

Selon un mode de réalisation du procédé selon l'invention, il est possible de recycler le flux gazeux (H) dans l'étape de réaction a), le flux (H) pouvant être riche en sulfure d'hydrogène en fonction de la quantité de sulfure d'hydrogène introduit au niveau de la réaction a). Ce flux peut être comprimé à l'aide d'un compresseur pour ensuite être introduit dans le réacteur de l'étape a). Une purge peut être présente sur cette ligne d'alimentation, afin d'éliminer d'éventuelles impuretés. Cette étape supplémentaire de recyclage permet également de diminuer la production de dioxyde de soufre généré par l'incinération de ces évents gazeux et permet de diminuer la quantité de sulfure d'hydrogène frais introduit lors de l'étape a).

De préférence, le 1,4-butanediol enrichi comprend entre 0,1% et 20% en poids de tétrahydrothiophène, de préférence entre 1% et 10% en poids de tétrahydrothiophène, plus préférentiellement entre 1% et 5% en poids de tétrahydrothiophène, par rapport au poids total du 1,4-butanediol enrichi.

### Etape g) - Recyclage

Le flux (I) comportant le 1,4-butanediol enrichi est conduit en tout ou partie vers l'étape a). Il est ainsi utilisé en tant que réactif. Il est introduit éventuellement en mélange avec du 1,4-butanediol frais. De préférence, la totalité du flux (I) est recyclée à l'étape a). De préférence, le flux (I) est engagé dans l'étape a), sans ajout de 1,4-butanediol frais.

Par 1,4-butanediol frais, on entend un 1,4-butanediol non enrichi au sens de la présente invention, c'est-à-dire un 1,4-butanediol n'ayant pas subi l'extraction gaz-liquide telle que selon l'invention.

Le flux (I) permet ainsi de recycler le tétrahydrothiophène et les autres composés soufrés, qui sont passés dans les évents gazeux. Ce flux permet également de recycler le tétrahydrofurane éventuellement présent dans les évents gazeux.

La présente invention concerne également un procédé de traitement des évents gazeux émis par une unité de production de tétrahydrothiophène à partir de 1,4-butanediol et de sulfure d'hydrogène comprenant les étapes suivantes :
- récupération des évents gazeux issus d'une unité de production de tétrahydrothiophène, lesdits évents gazeux comprenant au moins un composé soufré, de préférence le tétrahydrothiophène, et éventuellement du tétrahydrofurane ;
- réalisation d'une extraction gaz-liquide dudit au moins un composé soufré, de préférence le tétrahydrothiophène, par du 1,4-butanediol liquide de façon à obtenir un 1,4-butanediol liquide enrichi en composé(s) soufré(s), de préférence en tétrahydrothiophène; et éventuellement en tétrahydrofurane ;
- éventuellement utilisation dudit 1,4-butanediol liquide enrichi en tant que réactif pour la réaction de production de tétrahydrothiophène à partir de 1,4-butanediol et de sulfure d'hydrogène.

L'ensemble des éléments du procédé de traitement des évents gazeux sont ceux définis pour le procédé de production de tétrahydrothiophène selon l'invention.

### Description de la figure

La Figure 1 annexée est un schéma représentant une installation industrielle de production de tétrahydrothiophène selon l'invention.

L'étape a) de réaction est réalisée dans le réacteur R1 à partir de 1,4-butanediol enrichi, en tout ou partie, et de sulfure d'hydrogène.

Le flux d'alcool rentre par la conduite 1 dans le réacteur R1. Le flux de sulfure d'hydrogène rentre par la conduite 2 dans le réacteur R1. Le flux A sortant du réacteur R1 par la conduite 3 comprend du tétrahydrothiophène, de l'eau, éventuellement du sulfure d'hydrogène n'ayant pas réagi, éventuellement du tétrahydrofurane, éventuellement des sous-produits soufrés, éventuellement des oléfines, et éventuellement des incondensables.

L'étape b) de condensation est réalisée dans un dispositif R2, tel qu'un condenseur. Le dispositif R2 est alimenté par la conduite 3 en flux A. Le flux B comprenant le tétrahydrothiophène liquide est évacué du dispositif R2 par la conduite 4, les évents gazeux C sont évacués par la conduite 5.

L'étape c) de décantation est réalisée dans le dispositif R3. La phase aqueuse est séparée et éliminée par la conduite 6, le flux D comprenant le tétrahydrothiophène est séparé et évacué par la conduite 7 et les évents gazeux E sont éliminés par la conduite 8.

L'étape d) de distillation est réalisée dans le dispositif R4. Lorsqu'une colonne de type DWC est utilisée, les impuretés lourdes sont éliminées via une conduite 9, le tétrahydrothiophène est isolé via une conduite 10 et les impuretés légères F sont éliminées via une conduite 11.

L'étape f) d'extraction gaz-liquide est réalisée dans un dispositif R5. Le dispositif est alimenté par la conduite 12 en 1,4-butanediol liquide et par la conduite 13 en évents gazeux G. La conduite 13 récupère l'ensemble des évents et impuretés gazeux générés lors des étapes de séparation et purification du procédé, c'est-à-dire la conduite 5 récupérant les évents gazeux C issus de l'étape b) de condensation, la conduite 8 récupérant les évents gazeux E issus de l'étape c) de décantation et la conduite 11 récupérant la fraction gazeuse F issue de l'étape d) de distillation. En sortie de dispositif R5, les évents gazeux H sont éliminés par la conduite 14 et le flux I de 1,4-butanediol liquide enrichi notamment en tétrahydrothiophène est évacué par la conduite 15.

La conduite 15 amène le 1,4-butanediol enrichi vers le réacteur R1, afin qu'il soit utilisé en tant que réactif.

En fonction de la quantité d'évents gazeux H récupérés et de sa composition (selon si le sulfure d'hydrogène est introduit en excès dans la réaction a), il est possible de récupérer ces évents et de les réintroduire à l'étape a).

L'invention porte également sur l'utilisation d'un dispositif d'extraction gaz-liquide dans un procédé de production de tétrahydrothiophène, tel que défini ci-dessus pour la récupération et le recyclage des évents gazeux produits après purification du brut réactionnel.

Les exemples, qui suivent permettent d'illustrer la présente invention, mais ne sont en aucun cas limitatifs.

### Exemples

Le procédé selon l'invention a été mis en œuvre. L'étape d'extraction gaz-liquide a été réalisée dans une colonne d'adsorption IMTP Norton de diamètre 400mm et d'une hauteur de garnissage 4 m. La pression dans la colonne est de 3 bars absolus, avec une température de fond comprise entre 75 et 80 °C et une température de tête de 64°C. Le rapport massique des évents gazeux par rapport au 1,4-butanediol est de 1,13.

Les flux gazeux en entrée (flux entrant de la conduite 13 sur la figure 1) et en sortie (flux sortant de la conduite 14 sur la figure 1) du réacteur utilisé pour effectuer l'extraction gaz-liquide de l'étape f) du procédé ont été analysés. L'efficacité d'absorption a ainsi pu être calculée. Ces mesures figurent dans le tableau 1 ci-dessous.

**Tableau 1**

| | Efficacité Absorption |
|---|---|
| H₂S | 2.4% |
| CO₂ | 0.8% |
| H₂O | 90.8% |
| Oléfines en C₂ à C₄ | 0.9% |
| Mercaptans C1 à C4 | 15.4% |
| DHT/Thiophène | 37.1% |
| THF | 45.1% |
| THT | 94.8% |

Cet essai montre que 94.8% du THT présent dans les évents gazeux est absorbé par le BDO, et donc récupéré.

En fonction de la production de l'unité, si l'on considère que 38 kg/h de THT ont été récupérés, alors l'émission de 28 kg/h de SO₂ après incinération a été évitée, soit 224 tonnes/an (pour 8000 h/an de fonctionnement).

Un autre très grand avantage de cette absorption est la non-absorption des oléfines. En effet, plus de 99% des oléfines passent à travers l'absorbeur et seront quasiment éliminées par la purge de cette phase gazeuse sortante et ne seront pas recyclées dans la réaction.

Un autre avantage est aussi la non-absorption d'un incondensable (CO₂) issu des produits de craquage. En effet, l'absorption de cet incondensable par la colonne d'absorption au BDO aurait conduit à une accumulation d'incondensables rédhibitoire pour le procédé.

Concernant le THF, qui est l'intermédiaire de réaction, au moins 45% de ce produit est récupéré dans ces effluents, sachant que la teneur en THF dans ces effluents augmente avec le vieillissement du catalyseur.

Pour les autres composés absorbés en partie, leur recyclage va conduire à les retrouver dans les étapes de purification en aval de la réaction principale à part le sulfure d'hydrogène qui réagira au stade de la réaction principale.

## Revendications

1. Procédé de production de tétrahydrothiophène comprenant les étapes successives suivantes :
a) réaction de 1,4-butanediol en présence de sulfure d'hydrogène (H₂S), en phase gaz, en présence d'au moins un catalyseur pour former un flux (A) comprenant du tétrahydrothiophène, de l'eau, et éventuellement du sulfure d'hydrogène n'ayant pas réagi ;
b) condensation du flux (A) pour obtenir un flux (B) riche en tétrahydrothiophène et des évents gazeux (C) contenant les incondensables éventuellement formés à l'issue de l'étape a) ;
c) au moins une étape de purification, de préférence par décantation, du flux (B) avec séparation de la phase aqueuse, de la phase organique constituant le flux (D) et des évents gazeux (E) ;
d) éventuellement au moins une distillation du flux (D) pour isoler le tétrahydrothiophène des évents gazeux (F) ;
e) récupération du tétrahydrothiophène isolé à l'étape c) et éventuellement à l'étape d),
les étapes b) et c) pouvant être successives ou simultanées,
f) extraction gaz-liquide à l'aide d'une colonne, alimentée par un ou plusieurs flux gazeux (G) comprenant les évents gazeux (C) et/ou les évents gazeux (E) et éventuellement les évents gazeux (F) et par un flux liquide de 1,4-butanediol, pour former en sortie de colonne un flux gazeux (H) et un flux liquide (I) comprenant du 1,4-butanediol enrichi ;
g) recyclage de tout ou partie du flux (I) vers la réaction a).

2. Procédé selon la revendication 1, **caractérisé en ce que** le ratio massique des évents gazeux par rapport au 1,4-butanediol lors de l'étape f) est compris entre 0,001 et 10, de préférence entre 0,1 et 3, de manière encore préférée entre 0,5 et 2.

3. Procédé selon la revendication 1 ou 2, **caractérisé en ce que** l'extraction gaz-liquide est réalisée à une température comprise entre 25°C et 200°C, de préférence entre 25°C et 150°C, de préférence entre 25°C et 100°C, plus préférentiellement entre 40°C et 90°C.

4. Procédé selon l'une quelconque des revendications précédentes, **caractérisé en ce que** l'extraction gaz-liquide est réalisée à une pression comprise entre 1 et 40 bars absolus, de préférence entre 1 et 10 bars absolus, plus préférentiellement encore entre 1 et 5 bars absolus.

5. Procédé selon l'une quelconque des revendications précédentes, **caractérisé en ce que**, lors de l'étape g), la totalité du flux (I) est recyclée à l'étape a).

6. Procédé selon l'une quelconque des revendications précédentes, **caractérisé en ce que** le 1,4-butanediol enrichi comprend entre 0,1% et 20% en poids de tétrahydrothiophène, de préférence entre 1% et 10% en poids de tétrahydrothiophène, plus préférentiellement entre 1% et 5% en poids de tétrahydrothiophène, par rapport au poids total du 1,4-butanediol enrichi.

7. Procédé selon l'une quelconque des revendications précédentes, **caractérisé en ce que** les évents gazeux (C) issus de l'étape a) comprennent du tétrahydrothiophène, les incondensables éventuellement formés à l'issue de l'étape a), éventuellement le sulfure d'hydrogène n'ayant pas réagi, éventuellement du tétrahydrofurane, et éventuellement des sous-produits soufrés.

8. Procédé selon l'une quelconque des revendications précédentes, **caractérisé en ce que** l'extraction gaz-liquide est effectuée dans au moins une colonne d'absorption ou dans au moins une cuve, de préférence à agitation mécanique.

9. Procédé selon l'une quelconque des revendications précédentes, **caractérisé en ce que** le flux (H) provenant de l'étape d'extraction gaz-liquide est recyclé à l'étape a).

10. Utilisation d'un dispositif d'extraction gaz-liquide dans un procédé de production de tétrahydrothiophène, tel que défini à l'une quelconque des revendications 1 à 9 pour la récupération et le recyclage des évents gazeux produits après purification du brut réactionnel.

## Patentansprüche

1. Verfahren zur Herstellung von Tetrahydrothiophen, das nacheinander die folgenden Schritte umfasst:
a) Umsetzen von 1,4-Butandiol in Gegenwart von Schwefelwasserstoff (H₂S), in der Gasphase, in Gegenwart von mindestens einem Katalysator, um einen Stoffstrom (A) zu bilden, der Tetrahydrothiophen, Wasser und gegebenenfalls nicht umgesetzten Schwefelwasserstoff umfasst;
b) Kondensieren des Stoffstroms (A), um einen Stoffstrom (B), welcher reich an Tetrahydrothiophen ist, sowie Abgase (C) zu erhalten, welche die nicht kondensierbaren Verbindungen enthalten, wie sie gegebenenfalls nach Abschluss von Schritt a) gebildet wurden;
c) mindestens einen Schritt des Aufreinigens, vorzugsweise durch Absetzen, des Stoffstroms (B), wobei die wässrige Phase, die organischen Phase, welche den Stoffstrom (D) bildet, und Abgase (E) voneinander getrennt werden;
d) gegebenenfalls mindestens eine Destillation des Stoffstroms (D), um das Tetrahydrothiophen von den Abgasen (F) zu isolieren;
e) Gewinnen des Tetrahydrothiophens, welches im Schritt c) und gegebenenfalls im Schritt d) isoliert wurde,
wobei die Schritte b) und c) nacheinander oder gleichzeitig erfolgen können,
f) Gas-Flüssig-Extraktion mittels einer Kolonne, die von einem oder mehreren gasförmigen Stoffströmen (G), welche die Abgase (C) und/oder die Abgase (E) und gegebenenfalls die Abgase (F) umfassen, und von einem flüssigen Stoffstrom aus 1,4-Butandiol gespeist wird, um am Kolonnenauslass einen gasförmigen Stoffstrom (H) sowie einen flüssigen Stoffstrom (I) zu bilden, welcher angereichertes 1,4-Butandiol umfasst;
g) Rückführen der Gesamtheit oder eines Teils des Stoffstroms (I) zur Umsetzung a).

2. Verfahren nach Anspruch 1, **dadurch gekennzeichnet, dass** das Gewichtsverhältnis der Abgase gegenüber dem 1,4-Butandiol während des Schritts f) im Bereich von 0,001 bis 10, vorzugsweise von 0,1 bis 3, auf wiederum bevorzugte Weise von 0,5 bis 2 liegt.

3. Verfahren nach Anspruch 1 oder 2, **dadurch gekennzeichnet, dass** die Gas-Flüssig-Extraktion bei einer Temperatur im Bereich von 25 °C bis 200 °C, vorzugsweise von 25 °C bis 150 °C, vorzugsweise von 25 °C bis 100 °C, stärker bevorzugt von 40 °C bis 90 °C durchgeführt wird.

4. Verfahren nach einem beliebigen der vorhergehenden Ansprüche, **dadurch gekennzeichnet, dass** die Gas-Flüssig-Extraktion bei einem Druck im Bereich von 1 bis 40 bar absolut, vorzugsweise von 1 bis 10 bar absolut, noch stärker bevorzugt von 1 bis 5 bar absolut durchgeführt wird.

5. Verfahren nach einem beliebigen der vorhergehenden Ansprüche, **dadurch gekennzeichnet, dass** im Schritt g) der gesamte Stoffstrom (I) zum Schritt a) zurückgeführt wird.

6. Verfahren nach einem beliebigen der vorhergehenden Ansprüche, **dadurch gekennzeichnet, dass** das angereicherte 1,4-Butandiol zwischen 0,1 % und 20 % nach Gewicht an Tetrahydrothiophen, vorzugsweise zwischen 1 % und 10 % nach Gewicht an Tetrahydrothiophen, stärker bevorzugt zwischen 1 % und 5 % an Tetrahydrothiophen umfasst, bezogen auf das Gesamtgewicht des angereicherten 1,4-Butandiols.

7. Verfahren nach einem beliebigen der vorhergehenden Ansprüche, **dadurch gekennzeichnet, dass** die Abgase (C), welche aus dem Schritt a) stammen, Tetrahydrothiophen, die nicht kondensierbaren Verbindungen, wie sie gegebenenfalls nach Abschluss von Schritt a) gebildet wurden, gegebenenfalls nicht umgesetzten Schwefelwasserstoff, gegebenenfalls Tetrahydrofuran und gegebenenfalls schwefelhaltige Nebenprodukte umfassen.

8. Verfahren nach einem beliebigen der vorhergehenden Ansprüche, **dadurch gekennzeichnet, dass** die Gas-Flüssig-Extraktion in mindestens einer Absorptionskolonne oder in mindestens einem Behälter, vorzugsweise unter mechanischem Rühren, durchgeführt wird.

9. Verfahren nach einem beliebigen der vorhergehenden Ansprüche, **dadurch gekennzeichnet, dass** der Stoffstrom (H), welcher aus dem Schritt der Gas-Flüssig-Extraktion stammt, zum Schritt a) zurückgeführt wird.

10. Verwendung einer Vorrichtung zur Gas-Flüssig-Extraktion in einem Verfahren zur Herstellung von Tetrahydrothiophen, wie es in einem beliebigen der Ansprüche 1 bis 9 definiert ist, um Abgase, die nach dem Aufreinigen des Rohprodukts der Reaktion anfallen, zu gewinnen und zurückzuführen.

## Claims

1. Process for the production of tetrahydrothiophene comprising the following successive stages:
a) reaction of 1,4-butanediol in the presence of hydrogen sulfide (H₂S), in the gas phase, in the presence of at least one catalyst, to form a stream (A) comprising tetrahydrothiophene, water and possibly unreacted hydrogen sulfide;
b) condensation of the stream (A) to obtain a stream (B) rich in tetrahydrothiophene and vent gases (C) containing the noncondensables possibly formed upon completion of stage a);
c) at least one stage of purification, preferably by decanting, of the stream (B) with separation of the aqueous phase, of the organic phase constituting the stream (D) and of the vent gases (E);
d) optionally at least one distillation of the stream (D) to isolate the tetrahydrothiophene from the vent gases (F);
e) recovery of the tetrahydrothiophene isolated in stage c) and optionally in stage d),
it being possible for stages b) and c) to be successive or simultaneous,
f) gas-liquid extraction by means of a column, fed by one or more gas streams (G) comprising the vent gases (C) and/or the vent gases (E) and possibly the vent gases (F) and by a liquid stream of 1,4-butanediol, to form, at the column outlet, a gas stream (H) and a liquid stream (I) comprising enriched 1,4-butanediol;
g) recycling of all or part of the stream (I) to the reaction a).

2. Process according to Claim 1, **characterized in that** the ratio by weight of the vent gases with respect to the 1,4-butanediol during stage f) is of between 0.001 and 10, preferably between 0.1 and 3, more preferably between 0.5 and 2.

3. Process according to Claim 1 or 2, **characterized in that** the gas-liquid extraction is carried out at a temperature of between 25°C and 200°C, preferably between 25°C and 150°C, preferably between 25°C and 100°C, more preferentially between 40°C and 90°C.

4. Process according to any one of the preceding claims, **characterized in that** the gas-liquid extraction is carried out at a pressure of between 1 and 40 bar absolute, preferably between 1 and 10 bar absolute, more preferentially still between 1 and 5 bar absolute.

5. Process according to any one of the preceding claims, **characterized in that**, during stage g), the entire stream (I) is recycled in stage a).

6. Process according to any one of the preceding claims, **characterized in that** the enriched 1,4-butanediol comprises between 0.1% and 20% by weight of tetrahydrothiophene, preferably between 1% and 10% by weight of tetrahydrothiophene, more preferentially between 1% and 5% by weight of tetrahydrothiophene, with respect to the total weight of the enriched 1,4-butanediol.

7. Process according to any one of the preceding claims, **characterized in that** the vent gases (C) resulting from stage a) comprise tetrahydrothiophene, the noncondensables possibly formed upon completion of stage a), possibly unreacted hydrogen sulfide, possibly tetrahydrofuran and possibly sulfur-comprising byproducts.

8. Process according to any one of the preceding claims, **characterized in that** the gas-liquid extraction is carried out in at least one absorption column or in at least one tank, preferably with mechanical stirring.

9. Process according to any one of the preceding claims, **characterized in that** the stream (H) originating from the gas-liquid extraction stage is recycled in stage a).

10. Use of a gas-liquid extraction device in a process for the production of tetrahydrothiophene, as defined in any one of Claims 1 to 9, for the recovery and the recycling of the vent gases produced after purification of the crude reaction product.
